Europäisches Patentamt

European Patent Office

Office européen des brevets

(19)

(11) Numéro de publication : **0 358 539 B1**

# (12) FASCICULE DE BREVET EUROPEEN

(45) Date de publication du fascicule du brevet :
**08.07.92 Bulletin 92/28**

(51) Int. Cl.$^5$ : **C07C 31/40, C07C 29/124**

(21) Numéro de dépôt : **89402135.1**

(22) Date de dépôt : **27.07.89**

(54) **Procédé de préparation du trifluoroéthanol par hydrolyse, en phase gazeuse, du chlorotrifluoroéthane.**

(30) Priorité : **05.08.88 FR 8810813**

(43) Date de publication de la demande :
**14.03.90 Bulletin 90/11**

(45) Mention de la délivrance du brevet :
**08.07.92 Bulletin 92/28**

(84) Etats contractants désignés :
**AT BE CH DE ES FR GB GR IT LI LU NL SE**

(56) Documents cités :
**EP-A- 0 171 248**
**GB-A- 2 117 376**

(73) Titulaire : **RHONE-POULENC CHIMIE**
**25, quai Paul Doumer**
**F-92408 Courbevoie Cédex (FR)**

(72) Inventeur : **Doussain, Claude**
**2, rue Louis Girardet**
**F-69190 - Saint Fons (FR)**
Inventeur : **Gubelmann, Michel**
**39, Boulevard des Belges**
**F-69006 - Lyon (FR)**
Inventeur : **Tirel, Philippe-Jean**
**40, boulevard Kennedy**
**F-69600 - Oullins (FR)**

(74) Mandataire : **Fabre, Madeleine-France et al**
**RHONE-POULENC CHIMIE Service Brevets**
**Chimie 25, quai Paul Doumer**
**F-92408 Courbevoie Cédex (FR)**

## Description

La présente invention concerne un procédé de préparation du trifluoroéthanol par hydrolyse, en phase gazeuse, du chlorotrifluoroéthane.

Le trifluoro-2,2,2 éthanol (TFE) est un alcool trifluoré possédant une très bonne stabilité thermique, ce qui le rend apte à un certain nombre d'applications, notamment dans la synthèse d'anesthésiques fluorés, en pharmacologie en général et comme solvant.

On a décrit la préparation de cet alcool soit par hydrogénation de l'acide trifluoroacétique ou de ses esters, soit par hydrolyse de l'acétate de trifluoro-2,2,2 éthyle, en phase liquide dans un solvant comportant des groupes hydroxylés.

Ces divers procédés de préparation du TFE ne sont pas entièrement satisfaisants sur le plan industriel, de sorte qu'il a été engagé des recherches pour savoir s'il ne serait pas possible de réaliser l'hydrolyse directe, en phase gazeuse, du chloro-1 trifluoro-2,2,2 éthane.

C'est là le but de la présente invention.

On notera immédiatement que le succès de cette hydrolyse est surprenant, car on savait que le pouvoir électro-attracteur du groupement $CF_3$ de la molécule de chloro-1 trifluoro-2,2,2 éthane diminue, ou rend très difficile, la possibilité d'attaque de l'atome de carbone, lié au chlore, de cette molécule par un composé nucléophile.

Le procédé selon l'invention est que l'on admet sur un catalyseur solide constitué de, ou contenant au moins, un phosphate, un hydrogénophosphate ou un oxyde d'un métal di- ou trivalent, porté à une température supérieure à 350°C et de préférence comprise entre 400 et 500°C, un mélange gazeux de chloro-1 trifluoro-2,2,2 éthane et d'eau.

Les phosphates sont les sels (d'un métal di- ou trivalent, y compris les métaux des terres rares) de l'acide phosphorique. Les hydrogénophosphates sont les sels (d'un métal di- ou trivalent, y compris les métaux des terres rares) de l'acide phosphorique, dans lequel au moins un atome d'hydrogène a été remplacé par un atome de métal.

Les oxydes sont des produits connus.

Parmi ces catalyseurs, le plus intéressant est le phosphate de lanthane $LaPO_4$ ; ce phosphate de lanthane est un produit que l'on trouve dans le commerce et qui est d'une bonne pureté.

La température doit être d'au moins 350°C ; au-dessous de cette température, on ne décèle aucune réaction ; les essais ayant été effectués dans un réacteur en verre Pyrex, il n'a pas été jugé possible d'opérer à des températures supérieures à environ 500°C ; de toute façon, il semble qu'aux températures les plus hautes utilisées, on décèle l'existence de réactions parasites.

La réaction peut être réalisée en discontinu ou en continu, mais elle est beaucoup plus intéressante en continu. Pour cela, on fera passer le mélange gazeux sur le catalyseur porté à la température choisie et on recueillera ensuite les produits sortant par un simple piégeage à froid. Le temps de contact entre le mélange gazeux et le catalyseur peut être très court, il est généralement compris entre 1 et 10 s.

Enfin, les proportions relatives d'eau et de chloro-1 trifluoro-2,2,2 éthane sont comprises, en moles, entre 1 et 15. On pourra, si cela apparaît souhaitable, ajouter au mélange gazeux un gaz inerte, assurant une certaine dilution, tel que l'azote.

Les exemples non limitatifs ci-après illustrent l'invention.

Ces exemples ont été réalisés en suivant le protocole expérimental suivant :
– le solide catalytique (dont la granulométrie moyenne est de 1 mm) est introduit dans un réacteur tubulaire en verre Pyrex (ledit réacteur ayant un diamètre interne de 1,8 cm et une longueur totale de 16 cm).
– on dépose à la surface du lit du solide catalytique un lit de billes de verre,
– l'ensemble est chauffé pendant une demi-heure à la température de réaction choisie,
– puis on fait circuler dans le tube (en entrant du côté où se trouve le lit de billes de verre) le mélange des réactifs gazeux (eau et chloro-1 trifluoro-2,2,2 éthane). Lorsque l'équilibre réactionnel s'est établi, on piège les gaz sortant du réacteur et on procède par chromatographie en phase gazeuse à l'analyse des produits obtenus (produits dont la structure est confirmée par spectrométrie de masse).

Les résultats obtenus sont consignés dans le Tableau 1.

Ces résultats montrent que, à 490°C, on provoque, en une durée de l'ordre de 2 s, la transformation de 19 % du chloro-1 trifluoro-2,2,2 éthane pour obtenir 11 % (par rapport au chloro-1 trifluoro-2,2,2 éthane engagé dans le réacteur) de TFE, avec une sélectivité (RT), définie comme étant le nombre de moles de TFE obtenu par rapport au nombre de moles de chloro-1 trifluoro-2,2,2 éthane transformé, de 60 à 70 %.

2

EP 0 358 539 B1

TABLEAU 1

| Exemple | Catalyseur | Température (°C) | Rapport molaire $H_2O$/fluogène | Temps de contact (s) | Taux de conversion du chloro-1 tri-fluoro-2,2,2 éthane (%) | Rendement en TFE (%) | Sélectivité en TFE (%) |
|---|---|---|---|---|---|---|---|
| 1 | $LaPO_4$ | 350 | 2 | 2 | – | 1 | – |
| 2 | $LaPO_4$ | 400 | 2 | 2 | – | 2 | – |
| 3 | $LaPO_4$ | 450 | 2 | 2 | – | 4,9 | – |
| 4 | $LaPO_4$ | 450 | 2 | 3 | – | 3,4 | – |
| 5 | $LaPO_4$ | 450 | 5 | 2 | 13 | 7,6 | 59 |
| 6 | $LaPO_4$ | 450 | 10 | 1 | 11 | 6,1 | 56 |
| 7 | $LaPO_4$ | 490 | 5 | 2 | 19 | 11,7 | 62 |
| 8 | $LaPO_4$ | 490 | 5 | 2 | 19 | 11,3 | 60 |
| 9 | $LaPO_4$ | 490 | 10 | 1 | 19 | 8,7 | 46 |
| 10 | $(PO_4)_2(Ca)_3$ | 450 | 5 | 2 | – | 1 | – |

## Revendications

1. Procédé de préparation du trifluoroéthanol par hydrolyse du trifluorochloroéthane, caractérisé en ce que l'on admet sur un catalyseur solide constitué de - ou contenant - au moins un phosphate, un hydrogénophosphate ou un oxyde d'un métal di- ou trivalent, porté à une température supérieure à 350°C et de préférence comprise entre 400 et 500°C, un mélange gazeux de chloro-1 trifluoro-2,2,2 éthane et d'eau.

2. Procédé selon la revendication 1, caractérisé en ce que le temps de contact entre ledit mélange gazeux et ledit catalyseur solide est compris entre 1 et 10 s.

3. Procédé selon l'une des revendications 1 et 2, caractérisé en ce que le rapport molaire entre l'eau et le trifluoro-2 chloroéthane est compris entre 1 et 15.

4. Procédé selon l'une des revendications 1 à 4, caractérisé en ce que le catalyseur est le phosphate de lanthane.

## Patentansprüche

1. Verfahren zur Bereitung von Trifluorethanol durch Hydrolyse von Trifluorchlorethan, dadurch gekennzeichnet, daß ein Gasgemisch aus Chlor-1-trifluor-2,2,2-ethan und Wasser einem festen Katalysator zugeführt wird, der mindestens aus einem Phosphat, einem Hydrophosphat oder einem zwei- oder dreiwertigen Metalloxid besteht oder dies enthält, das auf eine Temperatur von über 350°C, vorzugsweise zwischen 400 und 500°C, gebracht wird.

2. Verfahren nach Anspruch 1, dadurch gekennzeichnet, daß die Berührungszeit zwischen dem Gasgemisch und dem festen Katalysator zwischen 1 und 10 s beträgt.

3. Verfahren nach einem der Ansprüche 1 und 2, dadurch gekennzeichnet, daß das Molverhältnis zwischen dem Wasser und dem Trifluor-2-chlorethan zwischen 1 und 15 beträgt.

4. Verfahren nach einem der Ansprüche 1 bis 4, dadurch gekennzeichnet, daß der Katalysator das Lanthanphosphat ist.

## Claims

1. A process for the preparation of trifluoroethanol by trifluorochloroethane hydrolysis, characterised in that a gaseous mixture of 1-chloro 2,2,2-trifluoroethane and water is added to a solid catalyst constituted of - or containing - at least one phosphate, a hydrogen phosphate or a di- or trivalent metal oxide raised to a temperature exceeding 350°C and preferably between 400 and 500°C.

2. A process according to Claim 1, characterised in that the contact time between said gaseous mixture and said solid catalyst is between 1 and 10 s.

3. A process according to one of Claims 1 and 2, characterised in that the molar ratio between the water and the 2-trifluoro chloroethane is between 1 and 15.

4. A process according to one of Claims 1 to 4, characterised in that the catalyst is lanthanum phosphate.